(19) Europäisches Patentamt
European Patent Office
Office européen des brevets

(11) Veröffentlichungsnummer: **0 340 706 B1**

(12) **EUROPÄISCHE PATENTSCHRIFT**

(45) Veröffentlichungstag der Patentschrift: **13.10.93**

(51) Int. Cl.[5]: **C07C 51/60**, C07C 53/42

(21) Anmeldenummer: **89107881.8**

(22) Anmeldetag: **29.04.89**

(54) **Verfahren zur Herstellung von Carbonsäurechloriden.**

(30) Priorität: **03.05.88 DE 3814969**

(43) Veröffentlichungstag der Anmeldung:
**08.11.89 Patentblatt 89/45**

(45) Bekanntmachung des Hinweises auf die Patenterteilung:
**13.10.93 Patentblatt 93/41**

(84) Benannte Vertragsstaaten:
**BE CH DE FR GB IT LI NL**

(56) Entgegenhaltungen:
**EP-A- 0 050 779**
**GB-A- 2 085 429**
**US-A- 3 318 950**

(73) Patentinhaber: **BASF Aktiengesellschaft**
**Carl-Bosch-Strasse 38**
**D-67063 Ludwigshafen(DE)**

(72) Erfinder: **Decker, Martin, Dr.**
**Maria-Stuart-Strasse 21**
**D-6700 Ludwigshafen(DE)**
Erfinder: **Franzischka, Wolfgang, Dr.**
**Flomersheimer Strasse 36**
**D-6710 Frankenthal(DE)**
Erfinder: **Irnich, Rudolf, Dr.**
**In den Hahndornen 2**
**D-6719 Bobenheim(DE)**
Erfinder: **Sauerwald, Manfred, Dr.**
**Im Langen Satz 2**
**D-6701 Meckenheim(DE)**

EP 0 340 706 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Diese Erfindung betrifft ein Verfahren zur Herstellung von Carbonsäurechloriden durch Umsetzung von Carbonsäuren oder deren Anhydriden mit Kohlenoxidchlorid in Gegenwart eines Carbonsäureamids.

Aus der BE-PS 620 385 und der DE-OS 2 057 956 ist bekannt, daß man Carbonsäuren mit Kohlenoxidchlorid in die entsprechenden Carbonsäurechloride überführen kann, wenn man die Umsetzung in Gegenwart von Dialkylcarbonsäureamiden als Katalysatoren durchführt.

Bei der technischen Herstellung der Carbonsäurechloride nach diesem Verfahren gibt es Schwierigkeiten, die in der EP-PS 31 504 näher erläutert sind. In dieser Patentschrift wird beschrieben, daß man die genannten Schwierigkeiten vermeidet und die Carbonsäurechloride auf technisch zufriedenstellende Weise erhält, wenn man als Dialkylformamid Diisobutylformamid als Katalysator verwendet.

Es wurde nun gefunden, daß man bei der Herstellung von Säurechloriden durch Umsetzung von Carbonsäuren oder deren Anhydriden mit Kohlenoxidchlorid in Gegenwart eines Carbonsäureamids erheblich bessere Ergebnisse erhält, wenn man als Carbonsäureamid eine Verbindung der Formel I verwendet

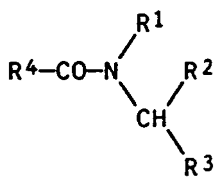

I,

in der $R^1$ einen Rest der Formeln

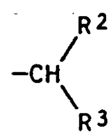

oder $-CH_2-R^5$,
$R^2$ einen Alkylrest mit 2 bis 9 C-Atomen, $R^3$ einen Alkylrest mit 1 bis 9 C-Atomen, $R^4$ und $R^5$ Wasserstoffatome oder Alkylreste mit 1 bis 9 C-Atomen bedeuten, und die beiden Reste $R^1$ und $R^2$ auch für einen Kohlenwasserstoffrest stehen können, der den Molekülteil

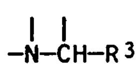

zu einem Heterocyclus vervollständigt, wobei die genannten Alkyl- und Kohlenwasserstoffreste noch Substituenten enthalten können, die unter den Umsetzungsbedingungen inert sind.

Die erfindungsgemäß zu verwendenden Carbonsäureamide, die im Unterschied zu den bereits verwendeten Dialkylamiden ein sekundäres Kohlenstoffatom

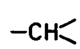

am Stickstoffatom tragen, ermöglichen überraschenderweise die Herstellung der Carbonsäurechloride in höherer Ausbeute und Reinheit. Ein weiterer unerwarteter Vorteil dieser Katalysatoren ist der, daß sie bei gegebener Produktionsanlage einen höheren Durchsatz erlauben. Sie entfalten außerdem eine gute Aktivität schon in sehr niedriger Konzentration, so daß die durch den Katalysator verursachten Nachteile, wie Feststoffausscheidung, Harzablagerung, Verunreinigung der Rohware durch stark gefärbte Zersetzungsprodukte des Katalysators, die bei Verwendung der bisher verwendeten Dialkylcarbonsäureamide aufgetreten sind, nicht oder nur in stark eingeschränkter Form in Erscheinung treten. Die erfindungsgemäß verwendeten Katalysatoren haben den weiteren Vorteil, daß sie auch bei einem Unterschuß oder bei einem sehr geringen Überschuß an Kohlenoxidchlorid noch eine hohe Reaktionsgeschwindigkeit bewirken, so daß das Verfahren

mit einem geringeren Energieaufwand durchgeführt werden kann.

In den erfindungsgemäß zu verwendenden Dialkylcarbonsäureamiden der Formel I weisen die Alkylreste, die geradkettig oder verzweigt sein können, 1 bis 9 C-Atome auf. Beispielsweise seien Methyl-, Ethyl-, Propyl-, iso-Propyl-, Butyl-, iso-Butyl-, Pentyl-, Hexylgruppen genannt. Sowohl die Alkylreste wie auch die durch $R^1$ und $R^2$ gebildeten Kohlenwasserstoffreste können unter den Umsetzungsbedingungen inerte Substituenten enthalten, z.B. Halogenatome, wie Chlor- oder Bromatome, Alkoxigruppen wie Methoxi- oder Ethoxigruppen, Alkoxicarbonylgruppen, wie Methoxicarbonyl- oder Ethoxicarbonylgruppen. Die Kohlenwasserstoffreste, die den Heterocyclus bilden, können außerdem noch Alkylgruppen, wie Methyl- oder Ethylgruppen enthalten. Als Heterocyclen kommen z.B. Pyrrolidin-, Piperidin- oder Hexamethyleniminringe in Betracht.

Bevorzugt sind solche Carbonsäureamide der Formel I, in denen die Alkylgruppen $R^3$ und $R^5$ 1 bis 4 C-Atome aufweisen. Von besonderem technischen Interesse ist die Verwendung von Carbonsäuredialkylamiden der Formel

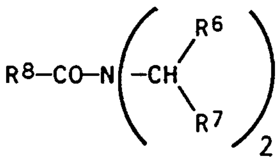

II,

in der $R^6$ einen Alkylrest mit 2 bis 4 C-Atomen, $R^7$ einen Alkylrest mit 1 bis 4 C-Atomen und $R^8$ ein Wasserstoffatom oder einen Alkylrest mit 1 bis 6 C-Atomen bedeuten. Beispielsweise seien die folgenden Dialkylcarbonsäureamide genannt.

Di-sec.-butylformamid, Di-3-pentylformamid, 3-Pentyl-isopropyl-formamid1-Formyl-2,6-dimethylpiperidin, 1-Formyl-2,5-dimethylpyrrolidin, 1-Propionyl-2,6-dimethylpiperidin.

Bevorzugte Ausgangsstoffe sind aliphatische Carbonsäuren oder deren Anhydride. Beispielsweise geht man von Carbonsäuren der allgemeinen Formel R-COOH aus, in der R einen Alkylrest mit 1 bis 18 Kohlenstoffatomen bedeutet. Der Alkylrest kann noch unter den Reaktionsbedingungen inerte Substituenten, wie Chloratome, Alkoxigruppen oder Alkoxicarbonylgruppen mit 1 bis 4 Kohlenstoffatomen enthalten. Folgende Ausgangsstoffe sind beispielsweise geeignet: Essigsäure, Mono-, Di-, Tri-chloressigsäure, Propionsäure, Buttersäure, Valeriansäure, Laurylsäure, Stearinsäure, Capronsäure, Isovaleriansäure, Palmitinsäure, 2-Ethylhexancarbonsäure, Methoxiessigsäure.

Die aliphatische Carbonsäure wird mit der stöchiometrischen Menge oder zweckmäßig mit einem Überschuß an Kohlenoxidchlorid, vorzugsweise in einem Verhältnis von 1,1 bis 1,5 Mol Kohlenoxidchlorid je Mol Ausgangssäure, umgesetzt. Das Carbonsäureamid der Formel I wendet man zweckmäßigerweise in einer Menge von 0,005 bis 1, vorzugsweise 0,02 bis 0,3 Mol.%, bezogen auf die Carbonsäure, an. Man kann auch größere Mengen an Carbonsäureamid, z.B. bis zu 5 Mol%, verwenden. Vorteilhaft wendet man jedoch eine möglichst geringe Menge an Carbonsäureamid an, da sie die weitere Verarbeitung der erhaltenen Carbonsäurechloride erleichtert. Die Umsetzung wird bei Temperaturen von 30 bis 150 °C, vorzugsweise 50 bis 100 °C, drucklos oder unter Druck und diskontinuierlich oder kontinuierlich durchgeführt.

Die Umsetzung wird bei der absatzweisen Herstellung von Säurechloriden beispielsweise so durchgeführt, daß man die flüssige oder geschmolzene Carbonsäure in einem geeigneten Rührbehälter vorlegt, das Carbonsäureamid darin löst und das Gemisch auf die gewünschte Reaktionstemperatur erwärmt. Bereits während des Aufheizens kann mit dem Einleiten von Kohlenoxidchlorid begonnen werden. Sobald die stöchiometrische Menge Kohlenoxidchlorid eindosiert ist, wird die pro Zeiteinheit zugeführte Menge auf 10 bis 30 % der ursprünglichen Menge zurückgenommen. Durch starken Kohlenoxidchlorid-Rückfluß wird das Ende der Reaktion angezeigt. Das Rohprodukt wird destillativ aufgearbeitet, wobei der Vorlauf des Carbonsäurechlorids das im Überschuß eingesetzte Kohlenoxidchlorid enthält. Dieser wird beim nachfolgenden Ansatz mit der vorgelegten Carbonsäure nach und nach zur Reaktion gebracht, sobald mit der Zufuhr von Kohlenoxidchlorid begonnen wird.

Bei der kontinuierlichen Arbeitsweise wird zunächst der Reaktor mit Carbonsäure und Katalysator bis nahe an den Überlauf gefüllt. Danach wird die Carbonsäure bei der gewünschten Reaktionstemperatur mit Kohlenoxidchlorid umgesetzt. Danach werden Carbonsäure, Carbonsäureamid und Kohlenoxidchlorid gleichzeitig und kontinuierlich dem Reaktor zugeführt. Der stündliche Volumenstrom der Carbonsäure, der im allgemeinen 10 bis 40 % des Reaktionsvolumens beträgt, kann, besonders bei höhermolekularen Carbonsäuren, auf 50 bis 80 % des Reaktorvolumens erhöht werden.

Das Carbonsäureamid wird kontinuierlich hinzugefügt. Es kann auch vorab in der Carbonsäure gelöst und mit dieser zusammen dem Reaktor zugeführt werden. Das Kohlenoxidchlorid kann bei der kontinuierlichen Arbeitsweise in der Menge auf wenige Mol.% über die stöchiometrische Menge begrenzt werden, weil in einer dem Reaktor nachgeschalteten Kolonne das überschüssige Kohlenoxidchlorid abgetrennt und sofort dem Reaktor wieder zugeführt wird.

Die nach dem Verfahren der Erfindung herstellbaren Verbindungen sind Lösungsmittel oder wertvolle Ausgangsstoffe für die Herstellung von Lösungsmitteln, Riechstoffen, Weichmachern, Waschmitteln, Peroxiden, Beizen und Schädlingsbekämpfungsmitteln. Bezüglich der Verwendung wird auf die genannten Veröffentlichungen und auf Ullmanns Encyklopädie der technischen Chemie, Band 5, Seite 100 ff. und 393 ff. verwiesen.

Beispiel 1

In einem mit Überlauf, Rückflußkühler und Zulaufeinrichtungen ausgestatteten Rührbehälter, dessen Fassungsvermögen bis zum Überlauf 300 Volumenteile beträgt, werden 144 Gewichtsteile 2-Ethylhexansäure und 0,31 Gewichtsteile (0,2 Mol.%) Di-sec.-butylformamid vorgelegt. Durch Einleiten von Kohlenoxidchlorid bei 70 °C wird die 2-Ethylhexansäure vollständig zum Carbonsäurechlorid umgesetzt. Die Temperatur des Kühlmittels im Rückflußkühler beträgt -70 °C. In das Reaktionsgemisch werden nun bei 70 °C stündlich 184 Gewichtsteile 2-Ethylhexansäure, in welcher 0,2 Mol.% Di-sec.-butylformamid gelöst sind, und 145 Gewichtsteile Kohlenoxidchlorid eingeleitet.

Aus dem Überlauf des Rührreaktors gelangt das rohe Reaktionsprodukt in einen Vorratsbehälter, aus welchem es der weiteren Verarbeitung zugeführt wird. Das rohe 2-Ethylhexansäurechlorid fällt 99,9 %ig und mit der Jodfarbzahl 80 an.

Beispiele 2 bis 4

In analoger Weise werden die in der Tabelle genannten Säuren unter den dort angegebenen Bedingungen in die Säurechloride übergeführt. Sofern das im Überschuß eingesetzte Kohlenoxidchlorid in der Aufarbeitungsstufe kondensiert und wieder der Reaktionsstufe zugeführt wird, erniedrigt sich die Zufuhr von frischem Kohlenoxidchlorid nahezu auf die stöchiometrische Menge.

In der Aufarbeitungsstufe wird das Reaktionsprodukt, sofern die Flüchtigkeit des Produktes es ermöglicht, destillativ gereinigt. Bei schwerflüchtigen Verbindungen kann die Reinigung mit Aktivkohle erfolgen. Wegen der guten Qualität der Rohprodukte erübrigt sich jedoch in vielen Fällen eine weitere Reinigung.

Wird im Beispiel 4 die Katalysatormenge schrittweise halbiert auf 0,1 Mol.% bzw. 0,05 u. 0,025 Mol.%, so erniedrigt sich die Jodfarbzahl des rohen Talgfettsäurechlorids schrittweise bis auf 30. Die Reaktionstemperaturen betragen hierbei 70 °C bzw. 80 und 100 °C.

Beispiel 5 (Vergleichsbeispiel)

In der in Beispiel 1 beschriebenen Apparatur werden auf entsprechende Weise 144 Gewichtsteile 2-Ethylhexansäure und 0,31 Gewichtsteile (0,2 Mol.%) Diisobutylformamid mit Kohlenoxidchlorid bei 70 °C vollständig zum Carbonsäurechlorid umgesetzt. In das Reaktionsgemisch werden nun bei 70 °C stündlich 81,5 Gewichtsteile 2 Ethylhexansäure, in welcher 0,2 Mol.% Diisobutylformamid gelöst sind, und 65 Gewichtsteile Kohlenoxidchlorid eingeleitet. Das dabei erhaltene Rohprodukt enthält 99,2 % 2-Ethylhexansäurechlorid und 0,2 % 2-Ethylhexansäureanhydrid. Es hat die Jodfarbzahl 150.

Beispiele 6 bis 8 (Vergleichsbeispiele)

In analoger Weise werden die in der Tabelle genannten Säuren unter den dort angegebenen Bedingungen in die Säurechloride übergeführt.

Tabelle

| Beispiel | Carbonsäure | Zulauf Säure | Zulauf $COCl_2$ | Reaktions-temperatur | Reinheit des Säurechlorids: Säurechlorid | Reinheit des Säurechlorids: Säure bzw. Anhydrid | Jodfarbzahl |
|---|---|---|---|---|---|---|---|
| | | [Gew.-Teile/h] | [Gew.-Teile/h] | [°C] | [Gew.-%] | [Gew.-%] | |
| 2 | Propionsäure | 83 | 120 | 60 | 99,0 | - | 60 |
| 3 | Isobuttersäure | 121 | 150 | 65 | 99,4 | - | 70 |
| 4 | Talgfettsäure | 117 | 48 | 70 | 99,8 | - | 60 |
| 6 | Propionsäure | 74 | 110 | 58 | 96,9 | 2,9 | 120 |
| 7 | Isobuttersäure | 88 | 110 | 55 | 93,8 | 5,5 | 80 |
| 8 | Talgfettsäure | 94 | 38 | 65 | 99,4 | - | 130 |

Beispiel 9

In einem Rührreaktor mit 500 Volumenteilen Fassungsvermögen werden analog Beispiel 9 273 Gewichtsteile Talgfettsäure und 0,19 Gewichtsteile (0,1 Mol.%) Di-3-pentylformamid vorgelegt, dann wird

bei 90°C mit Kohlenoxidchlorid umgesetzt. Man arbeitet entsprechend Beispiel 9 auf und erhält 292 Gewichtsteile rohes Talgfettsäurechlorid mit der Jodfarbzahl 20. Das Säurechlorid hat eine Reinheit von 99,8 %. Die Ausbeute beträgt 99,5 Mol.%.

Beispiel 10

In der im Beispiel 1 beschriebenen Apparatur werden 144 Gewichtsteile 2-Ethylhexansäure in Gegenwart von 0,185 Gewichtsteilen (0,1 Mol.%) Di-3-pentylformamid mit Kohlenoxidchlorid bei 80°C vollständig zum Säurechlorid umgesetzt. In das Reaktionsgemisch werden bei 80°C stündlich 271 Gewichtsteile 2-Ethylhexansäure, in der 0,348 Gewichtsteile Di-3-pentylformamid gelöst sind, und 205 Gewichtsteile Kohlenoxidchlorid eingeleitet. Das so erhaltene rohe 2-Ethylhexansäurechlorid hat eine Reinheit von 99,8 % und eine Jodfarbzahl von 40. Durch Destillation wird ein wasserhelles Produkt in praktisch quantitativer Ausbeute erhalten.

## Patentansprüche

1. Verfahren zur Herstellung von Säurechloriden durch Umsetzung von Carbonsäuren oder deren Anhydriden mit Kohlenoxidchlorid in Gegenwart eines Carbonsäureamids, dadurch gekennzeichnet, daß man als Carbonsäureamid eine Verbindung der Formel I verwendet

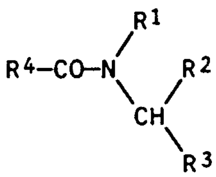

I,

in der $R^1$ einen Rest der Formeln

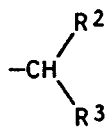

oder $-CH_2R^5$,
$R^2$ einen Alkylrest mit 2 bis 9 C-Atomen, $R^3$ einen Alkylrest mit 1 bis 9 C-Atomen, $R^4$ und $R^5$ Wasserstoffatome oder Alkylreste mit 1 bis 9 C-Atomen bedeuten, und die beiden Reste $R^1$ und $R^2$ auch für einen Kohlenwasserstoffrest stehen können, der den Molekülteil

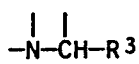

zu einem Heterocyclus vervollständigt, wobei die genannten Alkyl- und Kohlenwasserstoffreste noch Substituenten enthalten können, die unter den Umsetzungsbedingungen inert sind.

2. Verfahren nach Anspruch 1, dadurch gekennzeichnet, daß man ein Carbonsäureamid der Formel I verwendet, in dem die Alkylgruppen $R^3$ und $R^5$ 1 bis 4 C-Atome aufweisen.

**3.** Verfahren nach Anspruch 1, dadurch gekennzeichnet, daß man ein Carbonsäuredialkylamid der Formel

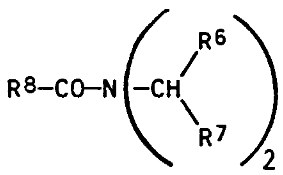

II,

in der $R^6$ einen Alkylrest mit 2 bis 4 C-Atomen, $R^7$ einen Alkylrest mit 1 bis 4 C-Atomen und $R^8$ ein Wasserstoffatom oder einen Alkylrest mit 1 bis 6 C-Atomen bedeutet, verwendet.

**4.** Verfahren nach Anspruch 1, dadurch gekennzeichnet, daß man die Umsetzung bei Temperaturen von 30 bis 150 ° C durchführt.

**5.** Verfahren nach Anspruch 1, dadurch gekennzeichnet, daß man die mindestens stöchiometrische Menge Kohlenoxidchlorid, bezogen auf die Carbonsäure, anwendet.

**6.** Verfahren nach Anspruch 1, dadurch gekennzeichnet, daß man das Carbonsäureamid der Formel I in einer Menge von 0,005 bis 1 Mol%, bezogen auf die Carbonsäure, anwendet.

**Claims**

**1.** A process for the preparation of an acyl chloride by reacting a carboxylic acid or its anhydride with carbonyl chloride in the presence of a carboxamide, wherein the carboxamide used is a compound of the formula I

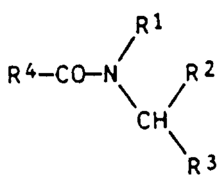

I

where $R^1$ is a radical of the formula

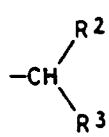

or $-CH_2-R^5$,
$R^2$ is alkyl of 2 to 9 carbon atoms, $R^3$ is alkyl of 1 to 9 carbon atoms, $R^4$ and $R^5$ are each hydrogen or alkyl of 1 to 9 carbon atoms and the two radicals $R^1$ and $R^2$ may furthermore be a hydrocarbon radical which completes the moiety.

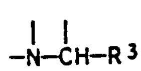

to form a heterocyclic structure, and the stated alkyl and hydrocarbon radicals may furthermore contain substituents which are inert under the reaction conditions.

2. A process as claimed in claim 1, wherein a carboxamide of the formula I, where the alkyl groups $R^3$ and $R^5$ are each of 1 to 4 carbon atoms, is used.

3. A process as claimed in claim 1, wherein a dialkylcarboxamide of the formula

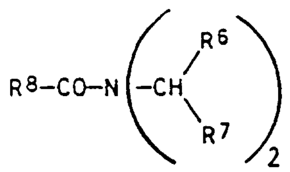

II

where $R^6$ is alkyl of 2 to 4 carbon atoms, $R^7$ is alkyl of 1 to 4 carbon atoms and $R^8$ is hydrogen or alkyl of 1 to 6 carbon atoms, is used.

4. A process as claimed in claim 1, wherein the reaction is carried out at from 30 to 150°C.

5. A process as claimed in claim 1, wherein not less than the stoichiometric amount, based on the carboxylic acid, of carbonyl chloride is used.

6. A process as claimed in claim 1, wherein the carboxamide of the formula I is used in an amount of from 0.005 to 1 mol %, based on the carboxylic acid.

**Revendications**

1. Procédé de préparation de chlorures d'acides par la réaction d'acides carboxyliques ou de leurs anhydrides avec le phosgène en présence d'un amide carboxylique, caractérisé en ce que, à titre d'amide carboxylique, on utilise un composé de la formule I

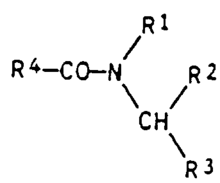

I

dans laquelle $R^1$ représente un radical de la formule

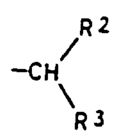

ou $-CH_2-R^5$,
$R^2$ représente un radical alkyle qui comporte de 2 à 9 atomes de carbone, $R^3$ représente un radical alkyle qui comporte de 1 à 9 atomes de carbone, $R^4$ et $R^5$ représentent des atomes d'hydrogène ou des radicaux alkyle qui comportent de 1 à 9 atomes de carbone et les deux radicaux $R^1$ et $R^2$ peuvent aussi représenter un reste hydrocarboné, qui complète la fraction de molécule

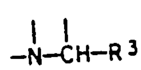

pour former un hétérocycle, où les radicaux alkyle et hydrocarboné peuvent encore contenir des substituants qui sont inertes dans les conditions réactionnelles.

2. Procédé suivant la revendication 1, caractérisé en ce que l'on utilise un amide carboxylique de la formule I dans lequel les radicaux alkyle $R^3$ et $R^5$ présentent de 1 à 4 atomes de carbone.

3. Procédé suivant la revendication 1, caractérisé en ce que l'on utilise un dialkylamide carboxylique de la formule

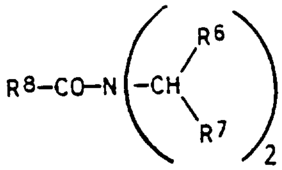

II

dans laquelle $R^6$ représente un radical alkyle qui comporte de 2 à 4 atomes de carbone, $R^7$ représente un radical alkyle qui comporte de 1 à 4 atomes de carbone et $R^8$ représente un atome d'hydrogène ou un radical alkyle qui comporte de 1 à 6 atomes de carbone.

4. Procédé suivant la revendication 1, caractérisé en ce que l'on réalise la réaction à des températures qui varient de 30 à 150 °C.

5. Procédé suivant la revendication 1, caractérisé en ce que l'on utilise au moins la proportion stoechiométrique de phosgène, par rapport à l'acide carboxylique.

6. Procédé suivant la revendication 1, caractérisé en ce que l'on utilise l'amide carboxylique de la formule I en une proportion de 0,005 à 1% molaire, par rapport à l'acide carboxylique.